# EUROPÄISCHE PATENTSCHRIFT

(11) **EP 2 489 494 B1**
(45) Veröffentlichungstag und Bekanntmachung des Hinweises auf die Patenterteilung: **28.06.2017**
(21) Anmeldenummer: 12153040.6
(22) Anmeldetag: 30.01.2012
(51) Int. Cl.: B29C 47/06, B29C 47/90, B29C 47/92, A61B 17/86, B29C 47/20, B29C 47/02, B29C 44/24, A61F 2/42

(54) **Verfahren zur Herstellung von Stäben und ihre Verwendung als Implantate**
Method for manufacturing rods and their use as implants
Procédé de fabrication de tiges et leur utilisation que les implants

(30) Priorität: 17.02.2011 DE 102011004305
(43) Veröffentlichungstag der Anmeldung: 22.08.2012
(73) Patentinhaber: Evonik Degussa GmbH, 45128 Essen (DE)
(72) Erfinder: Göring, Rainer, 46325 Borken (DE); Hartmann, Markus, 48324 Sendenhorst (DE); Kuhmann, Karl, 48249 Dülmen (DE); Kalthof, Bernfried, 63839 Kleinwallstadt (DE)

(56) Entgegenhaltungen:
- EP-A1- 0 987 033
- EP-A1- 1 741 540
- EP-A1- 2 062 717
- WO-A1-2009/045658
- DE-A1- 3 939 363
- JP-A- 2010 167 040
- US-A- 3 229 005
- US-A- 3 552 259
- US-A1- 2008 206 297

## Beschreibung

Erfindung betrifft ein Verfahren zur Extrusion von Stäben aus teilkristallinen Thermoplasten. Die Stäbe können kompakt sein; sie werden in diesem Fall als Vollstäbe bezeichnet. Ein Sonderfall hiervon sind Rundstäbe. Nach der EN ISO 15860 sind Rundstäbe extrudierte, gegossene oder gepresste längliche, gerade, massive Produkte mit einem über ihre gesamte Länge gleichbleibenden kreisförmigen Querschnitt.

Stäbe aus thermoplastischen Kunststoffen werden vorzugsweise für die spanende Herstellung von Fertigteilen verwendet, insbesondere von Fertigteilen mit geringeren Stückzahlen, Prototypenteilen oder Teilen, die mit Hilfe des Spritzgießverfahrens oder anderer Kunststoffverarbeitungsverfahren nur schwierig oder kaum hergestellt werden können.

Die Fertigung solcher Bauteile aus hochschmelzenden Thermoplasten wie Polyetheretherketon (PEEK) ähnelt aus Sicht des Anwenders eher der klassischen Metallverarbeitung: Standardisierte Halbzeuge wie Rohre, Profile oder Stäbe werden spanabhebend in die gewünschte Gestalt auf Maß gebracht. Entsprechend der klassischen Kunststofffertigung werden allein die Halbzeuge hergestellt: So werden Profile, Rohre oder Vollstäbe auf einer Extrusionsanlage prinzipiell ebenso extrudiert, wie dies bei der Herstellung von entsprechenden Halbzeugen aus PP oder PE üblich ist.

Stäbe mit kleineren Durchmessern kann man in guter Qualität durch konventionelle Extrusion einer thermoplastischen Formmasse erhalten. Bei Stäben ab einem Durchmesser je nach verwendeter Formmasse ab ca. 20 mm gestaltet sich dieses Verfahren jedoch schwierig, da infolge einer hohen Wärmeenergie, die von dem extrudierten Stab durch Kühlung abzuführen ist, eine ausreichende Formstabilität erst nach langer Kühlzeit erreicht werden kann, was sich dahingehend nachteilig auswirkt, dass der Stab infolge seines Eigengewichts in sich zusammensinken und sich damit der Querschnitt verändern kann oder dass es zu Durchbiegungen kommen kann. Ein Herabsetzen der Extrusionsgeschwindigkeit, um für den Stab verbesserte Abkühlbedingungen zu schaffen, erhöht die Fertigungskosten auf wirtschaftlich nicht mehr tragbare Werte.

In der Anmeldung DE 10 2004 015 072 A1 wird beschrieben, dass man bei der Extrusion im Werkzeug eine Kunststoffschmelze in zwei Ströme aufteilt, wobei aus der außen angeordneten Schmelze ein Rohr nach einem herkömmlichen Verfahren extrudiert wird. Die zweite Schmelze füllt das Rohr über eine Lanze. Damit das Rohr nicht kollabiert, wird Stützluft über den Kopf zugeführt und Unterdruck an der Kalibrierung angelegt. Es wird darauf hingewiesen, dass mit Hilfe dieses Verfahrens Rundstäbe gefertigt werden können, die ausgezeichnete optische Eigenschaften haben und im Querschnitt eine besonders hohe Maßhaltigkeit besitzen.

In der Anmeldung DE 37 18 036 A1 wird ein Verfahren zur Herstellung von Formteilen mit großen Querschnittsdimensionen, wie Stoßdämpfern oder Schutzabdeckungen aus Polyethylen, durch mehrschichtige Extrusion beschrieben. Hierbei wird ein erster Strom von geschmolzenem Material dazu verwendet, um ein Hohlprofil zu formen, das in einem Kalibriermantel gekühlt wird, während ein zweiter Strom von geschmolzenem Material in den Hohlraum extrudiert wird. Die Schmelze wird hierbei etwa dort injiziert, wo das Profil die Kalibrierung verlässt. Das äußere Hohlprofil, das sich verfestigt hat, aber noch eine gewisse Flexibilität aufweist, expandiert hierbei durch Bauchbildung, um so etwas mehr Materialvolumen aufzunehmen, als eigentlich erwünscht wäre. Durch die anschließende Schrumpfung beim Abkühlen wird der Bildung von Hohlräumen bzw. Lunkern entgegengewirkt.

Dieser Stand der Technik ist jedoch unbefriedigend. Die Anmeldung DE 10 2004 015 072 A1 bezieht sich ausschließlich auf amorphe Thermoplaste. Versucht man, auf diese Weise teilkristalline Thermoplaste zu verarbeiten, läuft man wegen der höheren Schwindung dieser Werkstoffe Gefahr, dass sich beim Abkühlen Hohlräume bilden, die für die Weiterverarbeitung nicht tolerierbar sind. Der Versuch der DE 37 18 036 A1, durch gezieltes Aufweiten der Bildung von Hohlräumen gegenzusteuern, ist dann nicht möglich, wenn es auf genaue Maßhaltigkeit ankommt. Weitere Verfahren zur Herstellung von Formteilen sind in den Druckschriften EP 1 741 540 A1 und EP 2 062 717 A1 offenbart. Die Aufgabe der Erfindung besteht darin, Stäbe aus teilkristallinen Thermoplasten herzustellen, die folgende Anforderungen erfüllen:
- Bei Stäben ab einem Durchmesser von etwa 20 mm sollte hinsichtlich der Maßhaltigkeit beim Durchmesser eine Verbesserung gegenüber konventioneller Extrusion erzielt werden. Im Idealfall sollten die Toleranzen, die durch die Normen DIN 16980 und EN ISO 15860 vorgegeben werden, weitestgehend erreicht oder unterschritten werden;
- die Abweichung des Stabes von der axialen Geraden, bezogen auf dessen Außenkanten und auf 1 Meter Länge, sollte ebenfalls möglichst gering sein. Die Abweichungen von der Geradheit können bei der spanenden Fertigung zu Problemen führen;
- ein Vollstab sollte weder Hohlräume noch Lunker besitzen, da diese bei der Weiterverarbeitung und für die Funktion des späteren Fertigteils sehr problematisch wären;
- ein Vollstab sollte darüber hinaus möglichst wenig innere Spannungen besitzen, um die spanende Bearbeitung zu erleichtern und den Verzug, der durch die Bearbeitung entstehen kann, zu reduzieren. Die inneren Spannungen können anhand des Verzugs von dünnen Scheiben beurteilt werden, die man vorsichtig aus den Vollstäben schneidet;
- schließlich sollte die Möglichkeit geschaffen werden, mehrschichtige Stäbe herzustellen, bei denen die Schichten aus unterschiedlichen Materialien bestehen.

Diese Aufgabe wurde durch ein Verfahren zur Herstellung von Stäben gelöst, bei dem
a) aus einer ersten Kunststoffformmasse, die die äußerste Schicht bildet und die zu mindestens 50 Gew.-%, vorzugsweise zu mindestens 60 Gew.-%, besonders bevorzugt zu mindestens 70 Gew.-% und insbesondere bevorzugt zu mindestens 80 Gew.-% aus einem teilkristallinen Thermoplasten besteht, ein Kunststoffprofil extrudiert wird,
b) innerhalb des Kalibrators das frisch extrudierte Profil mit einer zweiten Kunststoffformmasse gefüllt wird und
c) der neu gebildete Stab kalibriert, abgezogen und gekühlt wird, dadurch gekennzeichnet, dass die erste Kunststoffformmasse folgende Kennwerte aufweist:
   - Kristallitschmelzpunkt Tₘ von mindestens 170 °C, vorzugsweise mindestens 185 °C, besonders bevorzugt mindestens 200 °C und insbesondere bevorzugt mindestens 215 °C,
   - Kristallisationstemperatur Tₖ von maximal 70 K unterhalb von Tₘ , vorzugsweise von maximal 60 K unterhalb von Tₘ, besonders bevorzugt von maximal 55 K unterhalb von Tₘ und insbesondere bevorzugt von maximal 50 K unterhalb von Tₘ,
   - Schmelzenthalpie ΔH von mindestens 20 J/g, bevorzugt von mindestens 25 J/g, besonders bevorzugt von mindestens 30 J/g, insbesondere bevorzugt von mindestens 35 J/g und ganz besonders bevorzugt von mindestens 40 J/g als Maß für den Kristallisationsgrad,
   wobei Tₘ, Tₖ und ΔH gemäß ISO 11357 durch Aufheizen von Raumtemperatur bis maximal 390 °C bei einer Heizrate von 20 K/min, Abkühlen bis -60 °C bei einer Heizrate von 20 K/min und zweitem Aufheizen bis maximal 390 °C bei einer Heizrate von 20 K/min bestimmt werden. Tₘ und ΔH werden beim 2. Aufheizen bestimmt, Tₖ hingegen beim Abkühlen. Die maximale Aufheiztemperatur ist abhängig von der thermischen Stabilität sowie von Tₘ; es empfiehlt sich, hier mindestens um etwa 60 K über Tₘ zu gehen.

Der Kristallitschmelzpunkt Tₘ der ersten Kunststoffformmasse muss mindestens 170°C betragen, um eine ausreichend schnelle Verfestigung des extrudierten Profils zu gewährleisten. Diese Bedingung alleine reicht aber noch nicht aus. Bei verzögerter Kristallisation wäre das Profil nach dem Verlassen der Kalibrierung noch so flexibel, dass eine durch den Massedruck der Füllung hervorgerufene Aufweitung stattfinden würde. Aus diesem Grund muss zusätzlich die Kristallisation beim Abkühlen möglichst schnell einsetzen, also Tₖ möglichst hoch liegen. Der Kristallisationsgrad muss darüber hinaus ausreichend hoch sein, um die gewünschte Verfestigung zu gewährleisten. Dies lässt sich experimentell mit der Schmelzenthalpie korrelieren.

Die erfindungsgemäß hergestellten Stäbe können Rundstäbe sein; es kommen jedoch auch andere Geometrien in Frage, beispielsweise ovale, elliptische oder polygone (z. B. dreieckige, quadratische, rechteckige, rautenförmige, trapezförmige, fünfeckige oder sechseckige) Querschnitte. Im Falle eines Rundstabes ist das im Schritt a) extrudierte Profil ein Rohr. Das Profil kann einschichtig sein und somit ganz aus der ersten Kunststoffformmasse bestehen, oder es ist mehrschichtig, wobei zumindest die äußerste Schicht aus der ersten Kunststoffformmasse besteht.

Die erste Kunststoffformasse basiert auf einem teilkristallinen Thermoplasten, der beispielsweise ein Polyamid, ein teilaromatischer Polyester, ein Fluorpolymer (z. B. PFA, ETFE, EFEP, PVDF, PTFE), ein Polyphenylensulfid oder ein Polyarylenetherketon sein kann. Bei der Auswahl des Thermoplasten ist zu berücksichtigen, dass insbesondere die Kristallisationstemperatur Tₖ und die Schmelzenthalpie von den übrigen Bestandteilen der Formmasse beeinflusst werden. Tₖ kann beispielsweise durch Zusatz von Nukleierungsmitteln signifikant angehoben werden.

Polyamid ist herstellbar aus einer Kombination von Diamin und Dicarbonsäure, aus einer ω-Aminocarbonsäure oder dem entsprechenden Lactam. Grundsätzlich kann jedes ausreichend teilkristalline Polyamid verwendet werden, beispielsweise PA6, PA66 oder Copolyamide auf dieser Basis mit Einheiten, die sich von Terephthalsäure und/oder Isophthalsäure herleiten (im Allgemeinen als PPA bezeichnet) sowie PA9T und PA10T und deren Blends mit anderen Polyamiden. Geeignete Polyamide sind weiterhin beispielsweise PA610, PA88, PA8, PA612, PA810, PA108, PA9, PA613, PA614, PA812, PA128, PA1010, PA10, PA814, PA148, PA1012, PA11, PA1014, PA1212 und PA12. Selbstverständlich können auch hierauf basierende Copolyamide eingesetzt werden. Die Herstellung der Polyamide ist Stand der Technik.

Ebenso können auch Mischungen verschiedener Polyamide, ausreichende Verträglichkeit vorausgesetzt, verwendet werden. Verträgliche Polyamidkombinationen sind dem Fachmann bekannt; beispielsweise seien hier die Kombination PA12/PA1012, PA12/PA1212, PA612/PA12, PA613/PA12, PA1014/PA12 und PA610/PA12 sowie entsprechende Kombinationen mit PA11 aufgeführt. Im Zweifelsfall können verträgliche Kombinationen durch Routineversuche ermittelt werden.

Thermoplastische Polyester werden durch Polykondensation von Diolen mit Dicarbonsäuren bzw. deren polyesterbildenden Derivaten, wie Dimethylestern, hergestellt. Geeignete Diole haben die Formel HO-R-OH, wobei R einen divalenten, verzweigten oder unverzweigten aliphatischen und/oder cycloaliphatischen Rest mit 2 bis 18, vorzugsweise 2 bis 12, C-Atomen darstellt. Geeignete Dicarbonsäuren haben die Formel HOOC-R'-COOH, wobei R` einen divalenten aromatischen Rest mit 6 bis 20, vorzugsweise 6 bis 12, C-Atomen bedeutet.

Als Beispiel für Diole seien Ethylenglykol, Trimethylenglykol, Tetramethylenglykol, 2-Butendiol-1,4, Hexamethylenglykol, Neopentylglykol sowie Cyclohexandimethanol genannt. Die Diole können alleine oder als Diolgemisch eingesetzt werden.

Als aromatische Dicarbonsäuren kommen z. B. Terephthalsäure, Isophthalsäure, 1,4-, 1,5-, 2,6- bzw. 2,7-Naphthalindicarbonsäure, Biphenyl-4,4'-dicarbonsäure und Diphenylether-4,4'-dicarbonsäure in Frage. Bis zu 30 Mol-% dieser Dicarbonsäuren können durch aliphatische oder cycloaliphatische Dicarbonsäuren mit 3 bis 50 C-Atomen und bevorzugt mit 6 bis 40 C-Atomen, wie z. B. Bernsteinsäure, Adipinsäure, Sebacinsäure, Dodecandisäure oder Cyclohexan-1,4-dicarbonsäure, ersetzt sein.

Beispiele für geeignete Polyester sind Polypropylenterephthalat, Polybutylenterephthalat, Polyethylen-2,6-naphthalat, Polypropylen-2,6-naphthalat und Polybutylen-2,6-naphthalat.

Die Herstellung dieser Polyester gehört zum Stand der Technik (DE-OSS 24 07 155, 24 07 156; Ullmann's Enzyklopädie der technischen Chemie, 4. Auflage, Band 19, Seiten 65 ff., Verlag Chemie, Weinheim, 1980).

Das Fluorpolymer kann beispielsweise ein Polyvinylidenfluorid (PVDF), ein Ethylen-Tetrafluorethylen-Copolymer (ETFE), ein mit Hilfe einer Terkomponente wie beispielsweise Propen, Hexafluorpropen, Vinylfluorid oder Vinylidenfluorid modifiziertes ETFE (beispielsweise EFEP) oder ein Tetrafluorethylen-Perfluoralkylvinylether-Copolymer (PFA) sein.

### Polyphenylensulfid enthält Einheiten der Formel

(-C₆H₄-S-);

Vorzugsweise besteht es zu mindestens 50 Gew.-%, mindestens 70 Gew.-% bzw. mindestens 90 Gew.-% aus diesen Einheiten. Die restlichen Einheiten können solche sein, wie sie unten im Falle des Polyarylenetherketons angegeben sind, oder tri- oder tetrafunktionelle Verzweigereinheiten, die aus der Mitverwendung beispielsweise von Trichlorbenzol oder Tetrachlorbenzol bei der Synthese resultieren. Polyphenylensulfid ist in einer Vielzahl von Typen bzw. Formmassen handelsüblich.

### Polyarylenetherketon enthält Einheiten der Formeln

(-Ar-X-) und (-Ar'-Y-),

wobei Ar und Ar' einen zweiwertigen aromatischen Rest darstellen, vorzugsweise 1,4-Phenylen, 4,4'-Biphenylen sowie 1,4-, 1,5- oder 2,6-Naphthylen. X ist eine elektronenziehende Gruppe, bevorzugt Carbonyl oder Sulfonyl, während Y eine andere Gruppe wie O, S, CH₂, Isopropyliden oder dergleichen darstellt. Hierbei stellen mindestens 50 %, bevorzugt mindestens 70 % und besonders bevorzugt mindestens 80 % der Gruppen X eine Carbonylgruppe dar, während mindestens 50 %, bevorzugt mindestens 70 % und besonders bevorzugt mindestens 80 % der Gruppen Y aus Sauerstoff bestehen.

In der bevorzugten Ausführungsform bestehen 100 % der Gruppen X aus Carbonylgruppen und 100 % der Gruppen Y aus Sauerstoff. In dieser Ausführungsform kann das Polyarylenetherketon beispielsweise ein Polyetheretherketon (PEEK; Formel I), ein Polyetherketon (PEK; Formel II), ein Polyetherketonketon (PEKK; Formel III) oder ein Polyetheretherketonketon (PEEKK; Formel IV) sein, jedoch sind natürlich auch andere Anordnungen der Carbonyl- und Sauerstoffgruppen möglich.

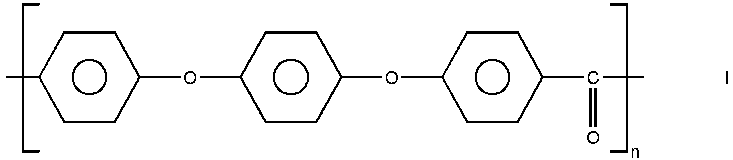

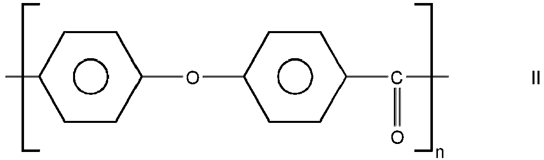

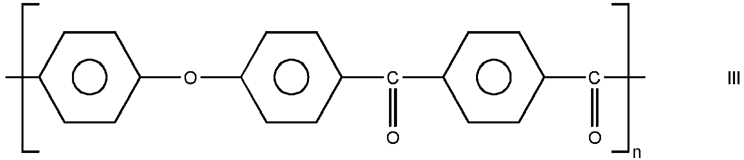

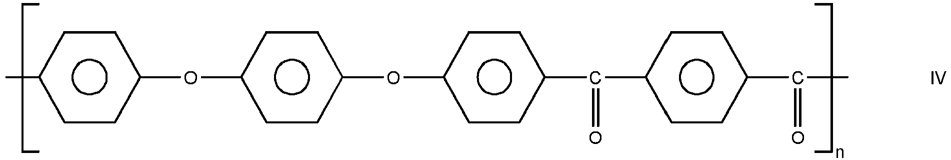

Das Polyarylenetherketon ist teilkristallin, was sich beispielsweise in der DSC-Analyse durch Auffinden eines Kristallitschmelzpunkts Tₘ äußert, der größenordnungsmäßig in den meisten Fällen um 300 °C oder darüber liegt.

Die Formmasse kann weitere Komponenten enthalten wie z.B. Schlagzähmodifikatoren, andere Thermoplaste, Weichmacher und andere übliche Zusatzstoffe, etwa Pigmente bzw. Füllstoffe wie Ruß, Titandioxid, Zinksulfid, Verstärkungsfasern wie z. B. Glasfasern, Kohlefasern oder Whiskers, Schmiermittel wie Graphit, Molybdändisulfid, Bornitrid oder PTFE, Nukleierungsmittel wie z.B. Talkum, Verarbeitungshilfsmittel wie Wachse, Zinkstearat oder Calciumstearat, Antioxidantien, UV-Stabilisatoren sowie Zusätze, die dem Produkt antielektrostatische Eigenschaften verleihen wie z. B. Kohlenstofffasern, Graphitfibrillen, Fasern aus rostfreiem Stahl bzw. Leitfähigkeitsruß.

In einer ersten Ausführungsform sind die erste und die zweite Formmasse identisch. In diesem Fall kann der Schmelzestrom vorteilhaft aufgeteilt werden, wie in der DE 10 2004 015 072 A1 beschrieben.

In einer zweiten Ausführungsform sind die erste und die zweite Formmasse nicht identisch, die zweite Formmasse fällt jedoch unter die gleiche anspruchsgemäße Definition wie die erste Formmasse. Dies ist insbesondere dann der Fall, wenn der zugrundeliegende Thermoplast beide Male identisch ist, die Formmassen sich jedoch in der Art bzw. Menge der übrigen Bestandteile unterscheiden. In anderen Fällen sind die beiden Thermoplaste verschieden, aber miteinander verträglich.

In einer dritten Ausführungsform fällt die zweite Formmasse nicht unter die gleiche anspruchsgemäße Definition wie die erste Formmasse. Sie kann teilkristallin oder amorph sein. Beispielhaft seien transparente Polyamide, Polypropylen, Polyethylenterephthalat, Polycarbonat, vollaromatische Polyester, Polyestercarbonat, Polysulfon, Polyethersulfon, Polyphenylsulfon, Polyphenylenether, Polyetherimid, Polyimid oder Blends aus Polyarylenetherketon mit gegebenenfalls größeren Anteilen Polysulfon, Polyethersulfon, Polyphenylsulfon, Polyetherimid und/oder Polyimid genannt. Die zweite Formmasse kann die üblichen Zusatzstoffe enthalten, wie sie weiter oben für die erste Formmasse bereits aufgeführt sind. Im Hinblick auf eine gute Anbindung der beiden Materialien ist eine ausreichende Haftungskompatibilität zwischen beiden Formmassen erforderlich.

In einer vierten Ausführungsform ist das im Schritt a) extrudierte Profil geschäumt. Das Schäumen kann chemisch durch Zusatz eines zersetzlichen Treibmittels oder physikalisch durch ein zudosiertes Treibgas erfolgen. Der Expansionsgrad, definiert durch das Verhältnis der Schaumdichte zur Dichte der ungeschäumten Formmasse, beträgt vorzugsweise 1,01 bis 10. Der Schaum kann offenzellig oder geschlossenzellig sein. Die vierte Ausführungsform kann mit der ersten, zweiten oder dritten Ausführungsform kombiniert werden.

In einer fünften Ausführungsform ist die im Schritt b) eingefüllte zweite Kunststoffformmasse geschäumt. Hier gilt das gleiche wie bei der vierten Ausführungsform. Die fünfte Ausführungsform kann mit der ersten, zweiten, dritten oder vierten Ausführungsform kombiniert werden.

Für die vorgesehene Anwendung ist es erwünscht, dass im fertigen Stab das im Schritt a) extrudierte Profil und der im Schritt b) eingefüllte Kern fest aufeinander haften. Hierzu müssen die beiden Formmassen ausreichend miteinander verträglich sein. In den Fällen, in denen eine ausreichende Verträglichkeit nicht gegeben ist, kann das Profil mehrschichtig coextrudiert werden, wobei die innerste Schicht aus einem geeigneten Haftvermittler besteht. Im Falle von PA12 (Profil) und PA6 (Kern) kann die Verträglichkeit beispielsweise durch eine Haftvermittlerschicht aus PA612 verbessert werden, während im Falle von PA612 (Profil) und Polyethylenterephthalat (PET; Kern) die Verträglichkeit beispielsweise mit einer Haftvermittlerschicht aus einem PA612/PET-Blend, das PA612-PET-Blockcopolymere enthält, hergestellt werden kann.

Um eine gute Haftung zu erzielen, sollte darüber hinaus die im Schritt b) eingefüllte Schmelze des Kernmaterials sich auf einem Temperaturniveau befinden, das mindestens im Bereich von Tₘ des Materials liegt, das die innere Oberfläche des extrudierten Profils bildet.

Der dem Extruder nachgeschaltete Kalibrator dient zur Formgebung der Außenschicht. Dabei kann z. B. eine typische Messing-Kalibrierung eingesetzt werden, wie sie in der Rohrextrusion verwendet wird. Die Kalibrierung kann je nach Wunsch sowohl intensiv gekühlt werden, um die Wärme schnell abzuführen, als auch beheizt werden, um durch ein langsameres Abkühlen die im Stab entstehenden Spannungen zu reduzieren. Mit Hilfe der Temperierung des Kalibrators empfiehlt es sich, die Temperatur des extrudierten Kunststoffprofils so einzustellen, dass eine feste Haftung der zweiten Formmasse gewährleistet ist. Dies ist vor allem bei geringen Wanddicken des Profils zu berücksichtigen.

Die zweite Kunststoffformasse wird als Schmelze an einer Stelle eingeführt, die vorzugsweise innerhalb etwa 1 % bis etwa 99 % der Kalibratorlänge liegt, besonders bevorzugt innerhalb etwa 10 % bis etwa 90 %, insbesondere bevorzugt innerhalb etwa 20 % bis etwa 80 % und ganz besonders bevorzugt innerhalb etwa 30 % bis etwa 70 %. In Sonderfällen, und zwar dann, wenn das extrudierte Profil eine vergleichsweise hohe Wandstärke besitzt und/oder die erste Kunststoffformmasse einen vergleichsweise hohen E-Modul und damit eine hohe Steifigkeit besitzt, kann die zweite Kunststoffformmasse auch erst nach dem Kalibrator eingeführt werden, ohne dass eine Aufweitung des Profils stattfindet. Unter diesen Voraussetzungen ist eine derartige Ausführungsform dem erfindungsgemäßen Verfahren äquivalent. Der Massedruck der zweiten Kunststoffformmasse liegt im Bereich von 2 bis 8 bar; er darf nur sehr geringe Schwankungen aufweisen, um produktionssicher lunkerfreie Stäbe herzustellen. Zur Herstellung von geschäumten Kernen kann der Druck geringer sein.

Dem Kalibrator nachgeschaltet kann eine Kühl- und/oder Temperstrecke mit unterschiedlichen Kühl- bzw. Heizmedien angeordnet sein. Dabei können ein Wasservollbad, Sprühbäder und/oder Gebläseluft eingesetzt werden. Insbesondere Stäbe mit größerem Durchmesser oder Vollstäbe aus fasergefüllten Formmassen werden temperiert bzw. methodisch abgekühlt, damit die inneren Spannungen nicht zu groß werden und es nicht aus diesem Grund zu einer vorzeitigen Zerstörung des Stabes kommt.

Die Abzugsgeschwindigkeit ist generell abhängig vom Durchmesser und von der verwendeten Formmasse. Kleinere Durchmesser werden mit höheren Geschwindigkeiten produziert.

Das erfindungsgemäße Verfahren kann mit einer flexiblen Kalibrierung kombiniert werden, so dass damit stufenlos unterschiedliche Stabdurchmesser produziert werden können. Derartige Kalibrierungen werden bereits bei der Extrusion von Rohren eingesetzt (z. B. WO 2004/091891, DE 10 2004 029 498 B3,
EP 1 627 724 A2, WO 00/16963 und WO 2005/018910). Die Verwendung einer flexiblen Kalibrierung ist wirtschaftlich hoch interessant, da bei der Fertigung von Bauteilen der Stab durch spanabhebende Verfahren auf das gewünschte Maß gebracht wird. Dabei ist es insbesondere bei hochwertigen Stäben aus teuren Formmassen wirtschaftlich relevant, möglichst wenig vom Außendurchmesser spanabhebend zu entfernen. Dies führt im Handel mit Vollstäben dazu, dass beispielsweise im Durchmesserbereich von ca. 5 mm bis ca. 40 mm die Durchmesser in 1 mm-Schritten angeboten und vielfach mit recht geringen Losgrößen hergestellt werden.

Im Rahmen der Erfindung können Stäbe mit einem Durchmesser (bzw. einem kleinsten Durchmesser in den Fällen, in denen kein runder Querschnitt vorliegt) von 15 bis 500 mm, vorzugsweise von 20 bis 400 mm und besonders bevorzugt von 25 bis 350 mm hergestellt werden. Die Wandstärke des im Schritt a) extrudierten Profils liegt hierbei üblicherweise im Bereich von 0,2 bis 25 % des Durchmessers, vorzugsweise im Bereich von 0,4 bis 20 % des Durchmessers, besonders bevorzugt im Bereich von 0,6 bis 16 % des Durchmessers und insbesondere bevorzugt im Bereich von 0,8 bis 14 % des Durchmessers. Falls kein runder Querschnitt vorliegt, ist mit "Durchmesser" der kleinste Durchmesser gemeint.

Im Rahmen der Erfindung sind mehrschichtige Stäbe und insbesondere mehrschichtige Rundstäbe von besonderem Interesse. Beispielsweise sind hier folgende Ausführungen möglich:
- Eine verschleißfeste oder antiabrasive Außenschicht und ein zäher Kern sind hervorragende Voraussetzungen für Anwendungen wie z. B. Zahnräder, Wellen oder Achsen, beispielsweise außen eine PEEK-Formmasse mit 30 % Kohlefasern oder mit jeweils 10 % Kohlefasern, Graphit und PTFE oder mit 30 % keramischem Füllstoff und innen eine unverstärkte PEEK-Formmasse.
- Bei einem Vollstab mit einem fasergefüllten Kernmaterial und einer nicht gefüllten Außenschicht können hohe Materialfestigkeit und glatte Oberflächen kombiniert werden.
- Eine weitere Möglichkeit besteht darin, eine Außenschicht mit guter Zerspanbarkeit zu wählen, beispielsweise aus dem gleichen Material wie der Kern, aber mit niedrigerem Molekulargewicht.
- Um eine Materialverbilligung zu erzielen, kann ein Vollstab aus einer hochwertigen Außenschicht (beispielsweise PEEK) und einer preisgünstigeren Kernkomponente bestehen, beispielsweise einem PEEK-Blend mit PFA, Polyimid, Polyetherimid, LCP wie beispielsweise flüssigkristallinem Polyester, Polysulfon, Polyethersulfon, Polyphenylsulfon, Polybenzimidazol (PBI) und/oder weiteren hochtemperaturbeständigen Polymeren.
- In einer weiteren Ausführungsform kann ein Stab aus einer harten kompakten Außenschicht und einem geschäumten Kern hergestellt werden. Hierbei wird in den zur Verfügung gestellten Rohrraum hineingeschäumt; anhand der gewählten Parameter wie Schmelzesteifigkeit, Treibmittelmenge, Art des Treibmittels usw. können der Expansionsgrad und die Zelligkeit auf bekannte Weise gesteuert werden. Auf diese Weise ist es möglich, einen gleichmäßigen Zellendurchmesser über den Querschnitt hinweg zu erhalten. Primäres Ziel ist hier die Gewichtsreduzierung, beispielsweise für Leichtbauanwendungen in der Luft- und Raumfahrt.
- Insbesondere für medizinische Anwendungen, beispielsweise für Implantate, sind auch die Kombination einer geschäumten Außenschicht mit einem harten bzw. zähen Kern interessant. Geschäumte Außenschichten verbessern die Angiogenese oder die Osseointegration, wobei gleichzeitig der feste, stabile Kern tragende Funktion hat. Offenzellige Schäume sind hierbei vorteilhaft. Eine mögliche Ausführungsform für medizinische Anwendungen ist ein aus einem anspruchsgemäßen Stab hergestelltes Teil mit einem kompakten Kern aus einer ungefüllten PEEK-Formmasse und einer Außenschicht aus einer offenzelligen geschäumten PEEK-Formmasse mit einem zahlenmittleren Zelldurchmesser von 1 µm bis 700 µm, vorzugsweise 10 µm bis 500 µm.
- Darüber hinaus können sowohl das vorextrudierte Rohr als auch der Kern geschäumt werden, z. B. dann, wenn unterschiedliche Schaumstrukturen gewünscht werden. Wenn bei Implantaten eine unterschiedliche Osseointegration benötigt wird oder möglich ist, kann auf diese Weise ein Implantat mit unterschiedlichen Zellstrukturen hergestellt werden, beispielsweise aus einer PEEK-Formmasse, um so eine gezielte Integration zu bewirken.

Gegenstand der Erfindung ist auch die Verwendung eines erfindungsgemäß hergestellten Stabs aus einer oder mehreren PEEK-Formmassen zur Herstellung eines Implantats.

Mit dem erfindungsgemäßen Verfahren kann beim extrudierten Profil eine ausreichende Formbeständigkeit schneller als beim Stand der Technik erreicht werden. Deshalb kann das Befüllen mit der zweiten Formmasse so weit vor dem Ende der Kalibrierung erfolgen, dass eine ausreichend lange Erstarrungszeit des Kernmaterials unter Massedruck gegeben ist, ohne dass eine Aufweitung des Profils erfolgt. Damit wird der Bildung von Lunkern wirkungsvoll entgegengewirkt. Der so erhaltene Stab ist maßhaltiger als Stäbe, die gemäß dem Stand der Technik hergestellt wurden.

### Beispiele

In den Beispielen und Vergleichsbeispielen wurden folgende Formmassen eingesetzt:
- PA12:: VESTAMID^{®} LX9030 gelb mit den Kennwerten Tₘ = 176 °C, Tₖ = 149 °C und ΔH = 64 J/g, jeweils mit der DSC-Methode gemäß ISO 11357 bestimmt;
- PEEK:: VESTAKEEP^{®} 4000G nf mit den Kennwerten Tₘ = 336 °C, Tₖ = 281 °C und ΔH = 42 J/g, jeweils mit der DSC-Methode gemäß ISO 11357 bestimmt.

### Vergleichsbeispiel 1:

Extrusion eines Vollstabs mit 65 mm Durchmesser aus PA 12 durch Standardextrusion. Die Versuchsbedingungen und das Ergebnis sind in der Tabelle 1 wiedergegeben.

### Beispiel 1:

Extrusion eines Vollstabs mit 65 mm Durchmesser aus PA12 mit einem Zweischichtwerkzeug; Dicke der Außenschicht 5 mm; die Lanze endet bei 70 % der Kalibratorlänge. Siehe Tabelle 1.

### Vergleichsbeispiel 2:

Extrusion eines Vollstabs mit 65 mm Durchmesser aus PEEK durch Standardextrusion; siehe Tabelle 2.

### Beispiel 2:

Extrusion eines Vollstabs mit 65 mm Durchmesser aus PEEK mit einem Zweischichtwerkzeug; Dicke der Außenschicht 5 mm; die Lanze endet bei 70 % der Kalibratorlänge. Siehe Tabelle 2.

**Tabelle 1: Versuche mit PA 12**

| | **Vergleichsbeispiel 1** | **Beispiel 1** |
|---|---|---|
| Temperaturprofil in Zylinder und Werkzeug | 200 °C, 220 °C, 240 °C, 240 °C, 240 °C | Beide Extruder hatten die gleichen Temperatureinstellungen, nämlich 200 °C, 220 °C, 240 °C, 240 °C, 240 °C |
| Schneckendrehzahl | 35 U/min (höhere Drehzahlen führten zu Druckschwankungen, bis hin zum Blockieren des Vollstabs in der Kalibrierung) | Extruder 1 für Außenschicht: 70 U/min |
| | | Extruder 2 für Kern: 120 U/min |
| Abzugsgeschwindigkeit | 0,5 m/h | 0,6 m/h |
| Massedruck | 2 - 4 bar. Höhere Drücke führten zu Problemen (siehe unter Schneckendrehzahl) | Extruder 1 (Außenschicht) deutlich größer als 4 bar |
| | | Extruder 2 ca. 5 bar zum Füllen des Rohres mit Schmelze |
| Vakuum | Keines | 0,1 - 0,3 bar (zur Fixierung der Außenschicht, damit das Rohr nicht kollabiert) |
| Vohstabdurchmesser: Abweichungen von der Rundheit im Durchschnitt | 2,0 mm | 0,4 mm |

**Tabelle 2: Versuche mit PEEK**

| | **Vergleichsbeispiel 2** | **Beispiel 2** |
|---|---|---|
| Temperaturprofil in Zylinder und Werkzeug | 320 °C, 340 °C, 350 °C, 360 °C, 360 °C | Beide Extruder hatten die gleichen Temperatureinstellungen, nämlich 320 °C, 340 °C, 350 °C, 360 °C, 360 °C |
| Schneckendrehzahl | 70 U/min | Extruder 1 für Außenschicht: 70 U/min |
| | | Extruder 2 für Kern: 60 U/min |
| Abzugsgeschwindigkeit | 1,68 m/h | 3,3 m/h |
| Massedruck | 2 - 4 bar. Drücke über 6 bar führten ohne eine anschließende Temperung zur Zerstörung des Vollstabs durch innere Spannungen | Extruder 1 deutlich größer als 4 bar |
| | | Extruder 2 ca. 5 bar |
| Vakuum | Keines | 0,2 - 0,48 bar |
| Vollstabdurchmesser: Abweichungen von der Rundheit im Durchschnitt | 1,8 mm | 0,4 mm |

## Patentansprüche

1. Verfahren zur Herstellung eines Stabes, bei dem
a) aus einer ersten Kunststoffformmasse, die die äußerste Schicht bildet und die zu mindestens 50 Gew.-% aus einem teilkristallinen Thermoplasten besteht, ein Kunststoffprofil extrudiert wird,
b) innerhalb des Kalibrators das frisch extrudierte Profil mit einer zweiten Kunststoffformmasse gefüllt wird und
c) der neu gebildete Stab kalibriert, abgezogen und gekühlt wird,
**dadurch gekennzeichnet,**
**dass** die erste Kunststoffformmasse folgende Kennwerte aufweist:
- Kristallitschmelzpunkt Tₘ gemäß ISO 11357 von mindestens 170 °C,
- Kristallisationstemperatur Tₖ gemäß ISO 11357 von maximal 70 K unterhalb von Tₘ und
- Schmelzenthalpie ΔH gemäß ISO 11357 von mindestens 20 J/g.

2. Verfahren gemäß Anspruch 1,
**dadurch gekennzeichnet,**
**dass** der Stab ein Rundstab ist.

3. Verfahren gemäß einem der vorhergehenden Ansprüche,
**dadurch gekennzeichnet,**
**dass** der Thermoplast der ersten Kunststoffformmasse ausgewählt ist aus der Gruppe Polyamid, teilaromatischer Polyester, Fluorpolymer, Polyphenylensulfid und Polyarylenetherketon.

4. Verfahren gemäß einem der vorhergehenden Ansprüche,
**dadurch gekennzeichnet,**
**dass** der Thermoplast der zweiten Kunststoffformmasse ausgewählt ist aus der Gruppe Polyamid, teilaromatischer Polyester, Fluorpolymer, Polyphenylensulfid, Polyarylenetherketon, transparentes Polyamid, Polypropylen, Polyethylenterephthalat, Polycarbonat, vollaromatischer Polyester, Polyestercarbonat, Polysulfon, Polyethersulfon, Polyphenylsulfon, Polyphenylenether, Polyetherimid, Polyimid und Blends aus Polyarylenetherketon mit Polysulfon, Polyethersulfon, Polyphenylsulfon, Polyetherimid und/oder Polyimid.

5. Verfahren gemäß einem der vorhergehenden Ansprüche,
**dadurch gekennzeichnet,**
**dass** die erste und die zweite Kunststoffformmasse identisch sind.

6. Verfahren gemäß einem der vorhergehenden Ansprüche,
**dadurch gekennzeichnet,**
**dass** das im Schritt a) extrudierte Kunststoffprofil geschäumt ist.

7. Verfahren gemäß einem der vorhergehenden Ansprüche,
**dadurch gekennzeichnet,**
**dass** die im Schritt b) eingefüllte zweite Kunststoffformmasse geschäumt ist.

8. Verfahren gemäß einem der vorhergehenden Ansprüche,
**dadurch gekennzeichnet,**
**dass** das im Schritt a) extrudierte Kunststoffprofil mehrschichtig ist, wobei die innerste Schicht aus einem Haftvermittler besteht.

9. Verfahren gemäß einem der vorhergehenden Ansprüche,
**dadurch gekennzeichnet,**
**dass** der Durchmesser bzw. der kleinste Durchmesser des Stabs im Bereich von 15 bis 500 mm liegt.

10. Verfahren gemäß einem der vorhergehenden Ansprüche,
**dadurch gekennzeichnet,**
**dass** die Wandstärke des im Schritt a) extrudierten Kunststoffprofils im Bereich von 0,4 bis 20 % des Durchmessers bzw. des kleinsten Durchmessers liegt.

11. Verwendung des gemäß einem der vorhergehenden Ansprüche hergestellten Stabs zur spanenden Herstellung von Fertigteilen.

12. Verwendung gemäß Anspruch 11,
**dadurch gekennzeichnet,**
**dass** der Stab aus einer oder mehreren Polyetheretherketon-Formmassen besteht und das Fertigteil ein Implantat ist.

13. Implantat, erhalten gemäß Anspruch 12.

14. Implantat gemäß Anspruch 13,
**dadurch gekennzeichnet,**
**dass** es einen ungeschäumten Kern und eine offenzellige geschäumte Außenschicht mit einem zahlenmittleren Zelldurchmesser von 1 bis 700 µm enthält.

## Claims

1. Process for producing a rod, by
a) extruding a plastics profile made of a first plastics moulding composition which forms the outermost layer and which is composed of at least 50% by weight of a semicrystalline thermoplastic,
b) within the calibrator, a second plastics moulding composition is inserted into the freshly extruded profile and
c) calibrating, drawing off and cooling the newly formed rod,
**characterized in that**
the first plastics moulding composition has the following properties:
- crystallite melting point Tₘ in accordance with ISO 11357 of at least 170°C,
- crystallization temperature Tₖ in accordance with ISO 11357 of at most 70 K below Tₘ and
- enthalpy of fusion AH in accordance with ISO 11357 of at least 20 J/g.

2. Process according to Claim 1,
**characterized in that**
the rod is a round rod.

3. Process according to either of the preceding claims,
**characterized in that**
the thermoplastic of the first plastics moulding composition is one selected from the group of polyamide, semiaromatic polyester, fluoropolymer, polyphenylene sulphide and polyarylene ether ketone.

4. Process according to any of the preceding claims, **characterized in that**
the thermoplastic of the second plastics moulding composition is one selected from the group of polyamide, semiaromatic polyester, fluoropolymer, polyphenylene sulphide, polyarylene ether ketone, transparent polyamide, polypropylene, polyethylene terephthalate, polycarbonate, fully aromatic polyester, polyester carbonate, polysulfone, polyether sulfone, polyphenyl sulfone, polyphenylene ether, polyether imide, polyimide and blends made of polyarylene ether ketone with polysulfone, polyether sulfone, polyphenyl sulfone, polyetherimide and/or polyimide.

5. Process according to any of the preceding claims, **characterized in that**
the first and the second plastics moulding composition are identical.

6. Process according to any of the preceding claims, **characterized in that**
the plastics profile extruded in step a) has been foamed.

7. Process according to any of the preceding claims, **characterized in that**
the second plastics moulding composition inserted in step b) has been foamed.

8. Process according to any of the preceding claims, **characterized in that**
the plastics profile extruded in step a) has a plurality of layers, where the innermost layer is composed of an adhesion promoter.

9. Process according to any of the preceding claims, **characterized in that**
the diameter or the smallest diameter of the rod is in the range from 15 to 500 mm.

10. Process according to any of the preceding claims, **characterized in that**
the wall thickness of the plastics profile extruded in step a) is in the range from 0.4 to 20% of the diameter or of the smallest diameter.

11. Use of the rod produced according to any of the preceding claims for producing finished parts by machining.

12. Use according to Claim 11,
**characterized in that**
the rod is composed of one or more polyether ether ketone moulding compositions and the finished part is an implant.

13. Implant obtained according to Claim 12.

14. Implant according to Claim 13,
**characterized in that**
it comprises an unfoamed core and an open-cell foamed external layer with a number-average cell diameter of from 1 to 700 µm.

## Revendications

1. Procédé pour la fabrication d'une tige, dans lequel
a) un profilé en matériau synthétique est extrudé à partir d'une première masse de moulage en matériau synthétique, qui forme la couche externe et qui est constituée, à raison d'au moins 50% en poids, par un thermoplastique partiellement cristallin,
b) le profilé fraîchement extrudé est rempli par une deuxième masse de moulage en matériau synthétique à l'intérieur du calibrateur et
c) la tige nouvellement formée est calibrée, retirée et refroidie,
**caractérisé en ce que** la première masse de moulage en matériau synthétique présente les caractéristiques suivantes .
- point de fusion des cristaux Tₘ selon la norme ISO 11357 d'au moins 170°C,
- température de cristallisation T_{c} selon la norme ISO 11357 d'au maximum 70 K sous Tₘ et
- enthalpie de fusion ΔH selon la norme ISO 11357 d'au moins 20 J/g.

2. Procédé selon la revendication 1, **caractérisé en ce que** la tige est une tige ronde.

3. Procédé selon l'une quelconque des revendications précédentes, **caractérisé en ce que** le thermoplastique de la première masse de moulage en matériau synthétique est choisi dans le groupe formé par le polyamide, le polyester partiellement aromatique, un polymère fluoré, le poly(sulfure de phénylène) et la polyarylénéthercétone.

4. Procédé selon l'une quelconque des revendications précédentes, **caractérisé en ce que** le thermoplastique de la deuxième masse de moulage en matériau synthétique est choisi dans le groupe formé par le polyamide, un polyester partiellement aromatique, un polymère fluoré, le poly(sulfure de phénylène), la polyarylénéthercétone, un polyamide transparent, le polypropylène, le poly(téréphtalate d'éthylène), le polycarbonate, un polyester complètement aromatique, le polyestercarbonate, la polysulfone, la polyéthersulfone, la polyphénylsulfone, le polyphénylénéther, le polyétherimide, le polyimide et les mélanges de polyarylénéthercétone et de polysulfone, de polyéthersulfone, de polyphénylsulfone, de polyétherimide et/ou de polyimide.

5. Procédé selon l'une quelconque des revendications précédentes, **caractérisé en ce que** la première et la deuxième masse de moulage en matériau synthétique sont identiques.

6. Procédé selon l'une quelconque des revendications précédentes, **caractérisé en ce que** le profilé en matériau synthétique extrudé dans l'étape a) est moussé.

7. Procédé selon l'une quelconque des revendications précédentes, **caractérisé en ce que** la masse de moulage en matériau synthétique injecté dans l'étape b) est moussée.

8. Procédé selon l'une quelconque des revendications précédentes, **caractérisé en ce que** le profilé en matériau synthétique extrudé dans l'étape a) est multicouche, la couche la plus interne étant constituée par un promoteur d'adhérence.

9. Procédé selon l'une quelconque des revendications précédentes, **caractérisé en ce que** le diamètre ou le plus petit diamètre de la tige se situe dans la plage de 15 à 500 mm.

10. Procédé selon l'une quelconque des revendications précédentes, **caractérisé en ce que** l'épaisseur de paroi du profilé en matériau synthétique extrudé dans l'étape a) se situe dans la plage de 0,4 à 20% du diamètre ou du plus petit diamètre.

11. utilisation de la tige fabriquée selon l'une quelconque des revendications précédentes pour la fabrication par usinage de pièces finies.

12. Utilisation selon la revendication 11, **caractérisée en ce que** la tige est constituée par une ou plusieurs masses de moulage à base de polyétheréthercétone et la pièce finie est un implant.

13. Implant, obtenu selon la revendication 12.

14. Implant selon la revendication 13, **caractérisé en ce qu'**il contient un noyau non moussé et une couche externe moussée à cellules ouvertes présentant un diamètre numérique moyen de cellule de 1 à 700 µm.
